(19) Europäisches Patentamt
European Patent Office
Office européen des brevets

(11) Veröffentlichungsnummer: **0 035 158**
**B1**

(12) # EUROPÄISCHE PATENTSCHRIFT

(45) Veröffentlichungstag der Patentschrift: **17.08.83**

(51) Int. Cl.³: **C 07 C 127/24**, C 08 G 18/78

(21) Anmeldenummer: **81101096.6**

(22) Anmeldetag: **17.02.81**

(54) **Verfahren zur Herstellung von Polyisocyanaten mit Biuretstruktur.**

(30) Priorität: **29.02.80 DE 3007679**

(43) Veröffentlichungstag der Anmeldung: **09.09.81 Patentblatt 81/36**

(45) Bekanntmachung des Hinweises auf die Patenterteilung: **17.08.83 Patentblatt 83/33**

(84) Benannte Vertragsstaaten: **BE DE FR GB IT**

(56) Entgegenhaltungen:
**US-A-3 124 605**
**Houben-Weyl (1968) Bd-X/4, S. 226**
**Houben-Weyl (1952) Bd VIII, S. 325**

(73) Patentinhaber: **BAYER AG, Zentralbereich Patente, Marken und Lizenzen, D-5090 Leverkusen 1, Bayerwerk (DE)**

(72) Erfinder: **Schwindt, Jürgen, Dr., Kleist-Platz 4, D-5090 Leverkusen 1 (DE)**
Erfinder: **Meyborg, Holger, Dr., Bergisch Gladbacher Strasse 1, D-5068 Odenthal (DE)**
Erfinder: **Reiff, Helmut, Dr., Walter-Flex-Strasse 16, D-5090 Leverkusen (DE)**
Erfinder: **König, Klaus, Dr., Heymannstrasse 50, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Verfahren zur Herstellung von Polyisocyanaten mit Biuretstruktur

Die vorliegende Erfindung betrifft ein neues Verfahren zur Herstellung von Polyisocyanaten mit Biuretstruktur durch Umsetzung von aliphatischen oder cycloaliphatischen Diisocyanaten mit unterschüssigen Mengen an bestimmten, nachstehend näher beschriebenen Aldoximen.

Polyisocyanate mit Biuretstruktur sind bekannt. Sie werden beispielsweise durch Umsetzung aromatischer oder aliphatischer Diisocyanate mit Wasser, Kristallwasser enthaltenden anorganischen Salzen oder Hydratwasser enthaltenden organischen Verbindungen (US-PS 3 124 605) oder mit Schwefelwasserstoff (DE-PS 1 165 580) über die Zwischenstufe eines Harnstoffdiisocyanats erhalten. Diese Verfahren des Standes der Technik sind mit dem Nachteil behaftet, daß sich stets unerwünschte, unlösliche, Harnstoffgruppen aufweisende Polyisocyanate bilden, die sich als Festkörper abscheiden und so beispielsweise die Apparaturen, die zur Herstellung der Biuretgruppen aufweisenden Polyisocyanate verwendet werden, verstopfen.

Es ist ferner bekannt, Polyisocyanate mit Biuretstruktur durch Umsetzung von aromatischen oder aliphatischen Diisocyanaten mit einwertigen tertiären Alkoholen herzustellen (US-PS 3 358 010). Nachteilhaft bei diesem Verfahren des Standes der Technik ist sowohl die erforderliche hohe Reaktionstemperatur von ca. 180 bis 200°C und insbesondere der Umstand, daß bei der Umsetzung leicht entzündbares iso-Butylen-Gas entsteht, das nur unter erheblichem Aufwand beseitigt werden kann.

Überraschenderweise wurden nun neue Biuretisierungsmittel gefunden, die die Herstellung von hochwertigen Polyisocyanaten mit Biuretstruktur unter Vermeidung der Nachteile der Verfahren des Standes der Technik gestatten. Der Begriff »Biuretisierungsmittel« wird im Rahmen der Erfindung für Verbindungen verwendet, die mit organischen Isocyanaten in der Hitze unter Bildung von Biuretgruppen aufweisenden Verbindungen reagieren. So sind beispielsweise Wasser, Schwefelwasserstoff oder tert.-Butanol die bei den Verfahren des obengenannten Standes der Technik eingesetzten »Biuretisierungsmittel«. Bei den neuen erfindungsgemäßen Biuretisierungsmitteln handelt es sich um Aldoximgruppen aufweisende organische Verbindungen. Bei ihrer Verwendung zur Herstellung von Biuretgruppen aufweisenden Polyisocyanaten auf Basis von aliphatischen oder cycloaliphatischen Diisocyanaten bilden sich überraschenderweise zu keinem Zeitpunkt der Reaktion unlösliche Harnstoffdiisocyanate, obwohl die erfindungsgemäße Umsetzung bei vergleichsweise niedrigen Temperaturen durchgeführt wird. Außerdem entstehen als Nebenprodukte neben leicht absorbierbarem Kohlendioxid lediglich die den Biuretisierungsmitteln entsprechenden Nitrile, d. h. leicht handhab- bzw. destillierbare Substanzen. Diese Nitrile entsprechen dem bezüglich der Biuretisierungsreaktion indifferenten Rest der eingesetzten Biuretisierungsmittel.

Gegenstand der vorliegenden Erfindung ist ein Verfahren zur Herstellung von Polyisocyanaten mit Biuretstruktur durch Umsetzung von aliphatischen oder cycloaliphatischen, Biuretgruppen-freien Diisocyanaten mit Verbindungen, die mit organischen Isocyanaten in der Hitze unter Bildung von Biuretgruppen aufweisenden Verbindungen reagieren, ansonsten jedoch gegenüber Isocyanatgruppen inert sind, als Biuretisierungsmittel, unter Verwendung von mindestens 2,5 Mol an Ausgangsdiisocyanat pro Grammäquivalent Biuretisierungsmittel, dadurch gekennzeichnet, daß man als Biuretisierungsmittel mindestens eine Aldoximgruppe aufweisende Verbindungen verwendet.

Erfindungsgemäß geeignete Biuretisuerungsmittel sind beliebige organische Verbindungen, die mindestens eine Aldoximgruppe aufweisen und ansonsten gegenüber Isocyanatgruppen inert sind. Besonders gut geeignete erfindungsgemäße Biuretisierungsmittel sind Verbindungen der Formel

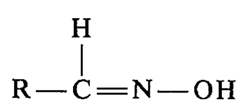

in welcher

R für einen Phenylrest, einen linearen oder verzweigten, gesättigten oder ungesättigten aliphatischen Kohlenwasserstoffrest mit 1 bis 6 Kohlenstoffatomen oder einen Rest der Formel

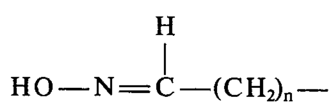

steht, wobei n für 0 oder eine ganze Zahl von 1 bis 5 steht.

Zu den bevorzugten erfindungsgemäßen Biuretisierungsmitteln gehören solche Verbindungen der genannten allgemeinen Formel, für welche R für einen gesättigten aliphatischen, gegebenenfalls verzweigten Kohlenwasserstoffrest mit 1 bis 3 Kohlenstoffatomen oder einen Rest der Formel

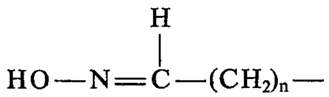

mit n = 3 oder 4 steht.

Typische Beispiele geeigneter Biuretisierungsmittel sind die Oxime bzw. Dioxime von Benzaldehyd, Ethanal, n-Propanal, n- und iso-Butanal, n-Pentanal, n-Hexanal, Acrolein, Malondialdehyd, Succindialdehyd, Glutardialdehyd oder Adipindialdehyd. Bevorzugte Biuretisierungsmittel sind die Oxime bzw. Dioxime von Acetaldehyd, n- und iso-Butanal, n-Propanal, Glutardialdehyd oder Adipinaldehyd. Gemäß einer besonderen Ausführungsform des erfindungsgemäßen Verfahrens kommen die genannten Aldoxime in Form ihrer Gemische mit bis zu 50 Mol-%, vorzugsweise 10 bis 30 Mol-% Wasser, bezogen auf Gemisch aus Biuretisierungsmittel und Wasser, zum Einsatz. Überraschenderweise treten bei Verwendung derartiger Gemische die obengenannten, bei der alleinigen Verwendung von Wasser als Biuretisierungsmittel beobachteten Schwierigkeiten nicht auf. Für das erfindungsgemäße Verfahren sind beliebige aliphatische oder cycloaliphatische Diisocyanate geeignet, insbesondere Verbindungen der Formel

$$Q(NCO)_2$$

in welcher

Q für einen gesättigten aliphatischen Kohlenwasserstoffrest mit 2 bis 18, vorzugsweise 6 bis 10 Kohlenstoffatomen, oder einen gegebenenfalls Alkyl-Substituenten und/oder Alkylen-Brücken aufweisenden cycloaliphatischen Kohlenwasserstoffrest mit insgesamt 6 bis 15, vorzugsweise 7 bis 13 Kohlenstoffatomen steht.

Typische Beispiele geeigneter Ausgangsdiisocyanate sind
Tetramethylendiisocyanat, Pentamethylendiisocyanat, Hexamethylendiisocyanat,
Decamethylendiisocyanat, 1,3-Cyclopentylendiisocyanat, 1,4-Cyclohexylendiisocyanat,
1,2-Cyclohexylendiisocyanat, Hexahydroxylendiisocyanat, Dichlorhexamethylendiisocyanat,
4,4'-Dicyclohexyldiisocyanat, 1,2-Di-(isocyanatomethyl)-cyclobutan,
1-Methyl-2,4-diisocyanatocyclohexan, 1-Methyl-2,6-diisocyanatocyclohexan,
4,4'-Diisocyanato-dicyclohexylmethan, 1,4-Diisocyanatocyclohexan und
1,3-Diisocyanatocyclohexan.

Bevorzugte Diisocyanate sind:
Hexamethylendiisocyanat, das Isomerengemisch aus
1-Methyl-2,4-diisocyanato-cyclohexan und 1-Methyl-2,6-diisocyanato-cyclohexan sowie
4,4'-Diisocyanato-dicyclohexylmethan.

Bei der Durchführung des erfindungsgemäßen Verfahrens kommen pro Grammäquivalent an im erfindungsgemäßen Biuretisierungsmittel vorliegenden Aldoximgruppen mindestens 2,5, vorzugsweise mindestens 3, besonders bevorzugt 6 bis 12 Mol des Ausgangsdiisocyanats zum Einsatz. Auch bei der Verwendung der erfindungsgemäßen Biuretisierungsmittel in Abmischung mit Wasser werden vorzugsweise diese Mengenverhältnisse zwischen Ausgangsdiisocyanat und Aldoximen eingehalten.

Das erfindungsgemäße Verfahren wird im allgemeinen im Temperaturbereich zwischen 70 und 180°C, vorzugsweise zwischen 70 und 160°C und besonders bevorzugt zwischen 120 und 150°C durchgeführt. Die Reaktionspartner können bei einer unterhalb dieser Temperatur liegenden Temperatur, beispielsweise Raumtemperatur, vorgemischt und anschließend auf die Reaktionstemperatur innerhalb der genannten Temperaturbereiche erhitzt werden. Es ist auch möglich, daß Ausgangsdiisocyanat auf eine Temperatur innerhalb der genannten Temperaturbereiche vorzuerhitzen und anschließend das Biuretisierungsmittel zuzudosieren. Das erfindungsgemäße Verfahren kann auch kontinuierlich, beispielsweise in Rührkesselkaskaden, durchgeführt werden. Je nach Siedepunkt des bei der Umsetzung entstehenden Nitrils wird dieses Nebenprodukt entweder während der Umsetzung fortlaufend abdestilliert oder nach der Umsetzung destillativ aus dem Reaktionsgemisch entfernt. Auch nicht umgesetztes Ausgangsdiisocyanat kann nach Beendigung der Umsetzung in bekannter Weise, beispielsweise unter Verwendung von Dünnschichtverdampfern, entfernt werden. Es ist selbstverständlich zweckmäßig, ein Biuretisierungsmittel einzusetzen, welches zu einem Nitril führt, dessen Siedepunkt sich vom Siedepunkt des Ausgangsdiisocyanats so unterscheidet, daß eine destillative Trennung des Nebenproduktes vom überschüssigen Ausgangsdiisocyanat möglich ist. Die Reaktionsansätze können nach Beendigung der Kohlendioxid-Entwicklung, gegebenenfalls auch nach einem kurzzeitigen Nacherhitzen auf eine Temperatur innerhalb der genannten Temperaturbereiche aufgearbeitet werden.

Die Mitverwendung von Katalysatoren ist beim erfindungsgemäßen Verfahren im allgemeinen nicht erforderlich. Zur Beschleunigung der Reaktion kämen, gewünschtenfalls, Säurekatalysatoren wie Bortrifluorid, Schwefelsäure, Phosphorsäure, phosphorige Säure oder Aluminiumtrichlorid in Betracht.

Die erfindungsgemäße Umsetzung wird im allgemeinen lösungsmittelfrei durchgeführt, kann jedoch auch in inerten Lösungsmitteln wie Dioxan, Tetrahydrofuran, Triethylenglykoldiacetat, Toluol, Benzol, Chlorbenzol, o-Dichlorbenzol, Butylacetat, Ethylenglykol-monoethylether-acetat oder Methylenchlorid vorgenommen werden.

Beim erfindungsgemäßen Verfahren werden stets praktisch farblose, klare, d. h. von unlöslichen Nebenprodukten freie Biuretpolyisocyanate erhalten. Die erfindungsgemäßen Verfahrensprodukte stellen wertvolle Ausgangsmaterialien zur Herstellung von Polyurethankunststoffen dar. Sie eignen sich insbesondere als Isocyanat-Komponente in Zweikomponenten-Polyurethanlacken.

Die nachfolgenden Beispiele dienen zur näheren Erläuterung des erfindungsgemäßen Verfahrens. Alle Prozentangaben beziehen sich auf Gewichtsprozente.

## Beispiel 1

In einer mit Heizmantel, Rührer, Thermometer, Tropftrichter und Aceton-Trockeneis-Kühlfalle ausgerüsteten Reaktionsapparatur werden 168 g (1 Mol) Hexamethylendiisocyanat vorgelegt und auf 120°C aufgeheizt. Bei dieser Temperatur werden 9,83 g (0,166 Mol) Acetaldoxim zugetropft. Unter lebhafter Kohlendioxidentwicklung steigt die Reaktionstemperatur hierbei auf 135 bis 145°C an. Das gleichzeitig abdestillierende Acetonitril wird in der Kühlfalle gesammelt. Die Zutropfgeschwindigkeit wird so geregelt, daß die Temperatur des Reaktionsgemisches 145°C nicht übersteigt. Nach Beendigung der Aldoximzugabe und der Kohlendioxidentwicklung wird das Reaktionsgemisch noch während 60 Minuten bei 130 bis 135°C gerührt. Anschließend wird überschüssiges Hexamethylendiisocyanat vom gebildeten Biuretpolyisocyanat durch Dünnschichtdestillation abgetrennt. Man erhält 74,8 g eines flüssigen, klaren, praktisch farblosen Biuretpolyisocyanats als Destillationsrückstand. Der NCO-Gehalt liegt bei 22,8%, der Monomerengehalt bei 0,67%.

## Beispiel 2

In der gleichen Apparatur wie in Beispiel 1 beschrieben werden bei Raumtemperatur 9,83 g Acetaldoxim mit 168 g Hexamethylendiisocyanat vermischt. Anschließend wird das Reaktionsgemisch unter Rühren allmählich erhitzt. Bei 90 bis 100°C beginnt die Entwicklung von Kohlendioxid. Ab 120°C wird die Reaktion unter starker Wärmetönung sehr lebhaft. Durch Kühlen wird die Reaktionstemperatur auf 125 bis 135°C gehalten, bis die Gasentwicklung beendet ist. Das zusammen mit dem Kohlendioxid dem Reaktionsgemisch entweichende Acetonitril wird in der Kühlfalle kondensiert. Durch Abdestillieren im Dünnschichtverdampfer von überschüssigem Hexamethylendiisocyanat werden 72 g eines flüssigen, klaren, praktisch farblosen Biuret-polyisocyanats als Destillationsrückstand erhalten.

Der NCO-Gehalt liegt bei 22,4%, der Monomerengehalt bei 0,58%.

## Beispiel 3

In der in Beispiel 1 beschriebenen Reaktionsapparatur werden 168 g (1 Mol) Hexamethylendiisocyanat vorgelegt und auf 130°C erhitzt. Anschließend tropft man unter Rühren ein Gemisch aus 7,37 g (0,125 Mol) Acetaldoxim und 0,75 g (0,041 Mol) Wasser zu. Nach Beendigung der Abspaltung von Kohlendioxid und Acetonitril, welches in der Kühlfalle gesammelt wird, wird das überschüssige Hexamethylendiisocyanat mittels Dünnschichter destillativ entfernt. Man erhält 74 g eines flüssigen, klaren, praktisch farblosen Biuretpolyisocyanats mit einem NCO-Gehalt von 23,1% und einem Monomerengehalt von 0,28%.

## Beispiele 4–14

Unter Verwendung der in Beispiel 1 beschriebenen Apparatur werden nach der in Beispiel 1 beschriebenen Verfahrensweise verschiedene Diisocyanate mit verschiedenen Biuretisierungsmitteln zur Reaktion gebracht. Die experimentellen Einzelheiten und die erhaltenen Ergebnisse werden in der nachstehenden Tabelle 1 zusammengefaßt. Die Monomerengehalte der Verfahrensprodukte liegen nach der Dünnschichtdestillation unter 0,7%.

Tabelle 1

| Beispiel | Diisocyanat | Biuretisierungsmittel | Zutropf-temp. (° C) | Biurettri-isocyanat % NCO | Nitril |
|---|---|---|---|---|---|
| 4 | 6 Mol HDI*) | 1 Mol Propanaldoxim | 135–140 | 23,2 | Propionitril |
| 5 | 6 Mol HDI | 0,67 Mol Propanaldoxim<br>0,33 Mol Wasser | 135–140 | 22,9 | Propionitril |
| 6 | 6 Mol HDI | 1 Mol Butyraldoxim | 140 | 22,7 | n-Butyronitril |
| 7 | 6 Mol HDI | 0,67 Mol Butyraldoxim<br>0,33 Mol Wasser | 140 | 23,1 | n-Butyronitril |
| 8 | 6 Mol HDI | 1 Mol iso-Butyraldoxim | 140 | 23,4 | iso-Butyronitril |
| 9 | 6 Mol HDI | 0,67 Mol Butyraldoxim<br>0,33 Mol Wasser | 140 | 23,4 | iso-Butyronitril |
| 10 | 12 Mol HDI | 1 Mol Glutardialdoxim | 145 | 22,4 | Glutarsäuredinitril |
| 11 | 24 Mol HDI | 0,85 Mol Glutardialdoxim<br>0,30 Mol Wasser | 145 | 22,3 | Glutarsäuredinitril |
| 12 | 12 Mol HDI | 1 Mol Adipindialdoxim | 145 | 22,8 | Adipinsäuredinitril |
| 13 | 12 Mol 4,4'-Diisocyanato-dicyclohexylmethan | 1 Mol Propanaldoxim | 140 | 14,8 | Propionitril |
| 14 | 12 Mol Isomerengemisch**) | 1 Mol Propanaldoxim | 140 | 21,3 | Propionitril |

*) Hexamethylendiisocyanat.
**) Isomerengemisch aus 1-Methyl-2,4-diisocyanatocyclohexan und 1-Methyl-2,6-diisocyanatocyclohexan.

**Patentansprüche**

1. Verfahren zur Herstellung von Polyisocyanaten mit Biuretstruktur durch Umsetzung von aliphatischen oder cycloaliphatischen, Biuretgruppen-freien Diisocyanaten mit Verbindungen, die mit organischen Isocyanaten in der Hitze unter Bildung von Biuretgruppen aufweisenden Verbindungen reagieren, ansonsten jedoch gegenüber Isocyanatgruppen inert sind, als Biuretisierungsmittel, unter Verwendung von mindestens 2,5 Mol an Ausgangsdiisocyanat pro Grammäquivalent Biuretisierungsmittel, dadurch gekennzeichnet, daß man als Biuretisierungsmittel mindestens eine Aldoximgruppe aufweisende Verbindungen verwendet.

2. Verfahren gemäß Anspruch 1, dadurch gekennzeichnet, daß man als Biuretisierungsmittel Aldoxime der Formel

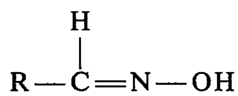

verwendet, wobei

R für einen Phenylrest, einen linearen oder verzweigten, gesättigten oder ungesättigten aliphatischen Kohlenwasserstoffrest mit 1 bis 6 Kohlenstoffatomen oder einen Rest der Formel

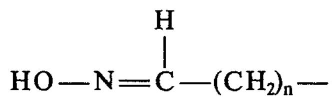

bedeutet, in welcher n für 0 oder eine ganze Zahl von 1 bis 5 steht.

3. Verfahren gemäß Anspruch 1 und 2, dadurch gekennzeichnet, daß man pro Grammäquivalent an im Biuretisierungsmittel vorliegenden Aldoximgruppen mindestens 2,5 Mol Diisocyanat verwendet.

4. Verfahren gemäß Anspruch 1 bis 3, dadurch gekennzeichnet, daß man die Umsetzung bei einer Temperatur zwischen 70 und 180° C durchführt.

5. Verfahren gemäß Anspruch 1 bis 4, dadurch gekennzeichnet, daß man das Biuretisierungsmittel in Form seines Gemisches mit bis zu 50 Mol-%, bezogen auf das Gemisch aus Biuretisierungsmittel und Wasser, an Wasser verwendet.

6. Verfahren gemäß Anspruch 1 bis 5, dadurch gekennzeichnet, daß man als Biuretisierungsmittel die Oxime von Ethanal, n-Propanal, n- und iso-Butanal oder die Dioxime von Glutardialdehyd oder Adipindialdehyd verwendet.

**Claims**

1. A process for the production of polyisocyanates having a biuret structure by reacting aliphatic or cycloaliphatic diisocyanates free from biuret groups with compounds which react with organic isocyanates at elevated temperature to form compounds containing biuret groups but which are otherwise inert to isocyanate groups as biuretising agents using at least 2.5 moles of starting diisocyanate per gram equivalent of biuretising agent, characterised in that compounds containing at least one aldoxime group are used as the biuretising agent.

2. A process as claimed in Claim1, characterised in that aldoximes corresponding to the following formula

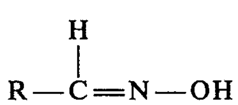

in which

R represents a phenyl radical, a linear or branched, saturated or unsaturated aliphatic hydrocarbon radical containing from 1 to 6 carbon atoms or a radical of the following formula

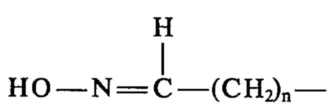

wherein n = 0 or is an integer of from 1 to 5 are used as the biuretising agent.

3. A process as claimed in Claims 1 and 2, characterised in that at least 2.5 moles of diisocyanate are

used per gram equivalent of aldoxime groups present in the biuretising agent.

4. A process as claimed in Claims 1 to 3, characterised in that the reaction is carried out at a temperature of from 70 to 180° C.

5. A process as claimed in Claims 1 to 4, characterised in that the biuretising agent is used in the form of a mixture with up to 50 mole percent of water, based on the mixture of biuretising agent and water.

6. A process as claimed in Claims 1 to 5, characterised in that the oximes of ethanal, n-propanal, n- and iso-butanal or the dioximes of glutardialdehyde or adipic dialdehyde are used as the biuretising agent.

## Revendications

1. Procédé pour la fabrication de polyisocyanates à structure de biuret par réaction de diisocyanates aliphatiques ou cycloaliphatiques exempts de groupes biuret avec des composés qui réagissent à chaud avec les isocyanates organiques avec formation de composés à groupes biuret, mais sinon sont inertes vis-à-vis des groupes isocyanate, comme agents de biurétisation, avec utilisation d'au moins 2,5 moles de diisocyanate de départ par équivalent-gramme d'agents de biurétisation, caractérisé en ce que l'on utilise comme agents de biurétisation des composés présentant au moins un groupe aldoxime.

2. Procédé selon la revendication 1, caractérisé en ce que l'on utilise comme agents de biurétisation des aldoximes de formule

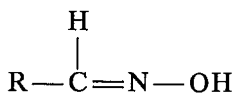

dans laquelle

R représente un reste phényle, un reste d'hydrocarbure aliphatique saturé ou insaturé, linéaire ou ramifié, en $C_1-C_{16}$ ou un reste de formule

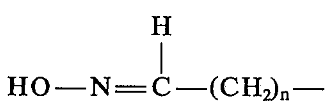

dans laquelle n est égal à 0 ou un nombre entier de 1 à 5.

3. Procédé selon la revendication 1 ou 2, caractérisé en ce que l'on utilise au moins 2,5 moles de diisocyanate par équivalent-gramme de groupes aldoximes présents dans l'agent de biurétisation.

4. Procédé selon les revendications 1 à 3, caractérisé en ce que l'on effectue la réaction à une température comprise entre 70 et 180° C.

5. Procédé selon les revendications 1 à 4, caractérisé en ce que l'on utilise l'agent de biurétisation sous forme de son mélange avec jusqu'à 50 moles% d'eau, par rapport au mélange d'agents de biurétisation et d'eau.

6. Procédé selon les revendications 1 à 5, caractérisé en ce que l'on utilise comme agent de biurétisation les oximes de l'éthanal, du n-propanal, du n- ou iso-butanal ou les dioximes du dialdéhyde glutarique ou du dialdéhyde adipique.